(19) Europäisches Patentamt
European Patent Office
Office européen des brevets

(11) Veröffentlichungsnummer: **0 314 620**
**A1**

# (12) EUROPÄISCHE PATENTANMELDUNG

(21) Anmeldenummer: **88810714.1**

(51) Int. Cl.4: **C 07 D 249/20**

(22) Anmeldetag: **19.10.88**

(30) Priorität: **29.10.87 CH 4230/87**

(43) Veröffentlichungstag der Anmeldung:
**03.05.89 Patentblatt 89/18**

(84) Benannte Vertragsstaaten:
**CH DE ES FR GB IT LI**

(71) Anmelder: **CIBA-GEIGY AG**
**Klybeckstrasse 141**
**CH-4002 Basel (CH)**

(72) Erfinder: **Burdeska, Kurt, Dr.**
**Laufenburgerstrasse 30**
**CH-4058 Basel (CH)**

**Göttel, Otto Richard, Dr.**
**Rte du Centre 23**
**CH-1723 Marly (CH)**

(54) **Verfahren zur Herstellung von 3-[2'H-Benzotriazol-(2')-yl]-4-hydroxybenzolsulfonsäuren und deren Salzen.**

(57) Es wird ein Verfahren zur Herstellung von 3-[2'H-Benzotriazol-(2')-yl]-4-hydroxybenzolsulfonsäuren und deren Salzen der Formel

(1)

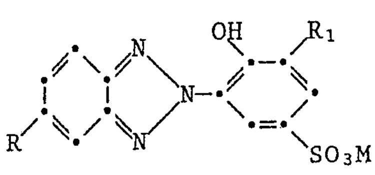

worin
R Wasserstoff oder Chlor,
$R_1$ verzweigtes oder geradkettiges $C_1$-$C_{12}$-Alkyl oder Phenyl-$C_1$-$C_3$-alkyl und
M Natrium oder Kalium bedeuten,
durch Sulfierung von einem 2-(2'-Hydroxy-5'-tert.-butylphenyl)-benzotriazol der Formel

(2)

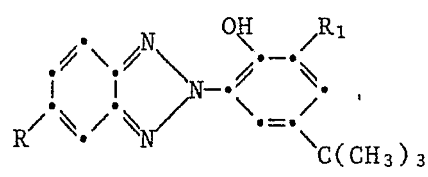

worin R und $R_1$ die oben angegebene Bedeutung haben und Neutralisierung des Endproduktes auf pH 7 beschrieben. Bei der Sulfierung wird die tert.-Butylgruppe unter Abspaltung von Isobutylen durch die Gruppe $-SO_3M$ ersetzt.

EP 0 314 620 A1

**Beschreibung**

## Verfahren zur Herstellung von 3-[2′H-Benzotriazol-(2′)-yl]-4-hydroxybenzolsulfonsäuren und deren Salzen

Die vorliegende Erfindung betrifft ein Verfahren zur Herstellung von 3-[2′H-Benzotriazol-(2′)-yl]-4-hydroxybenzolsulfonsäuren un deren Salzen.

Natriumsalze von 3-[2′H-Benzotriazol-(2′)-yl]-4-hydroxybenzolsufonsäuren sind z.B. aus EP-A-0 112 120 bekannt. Sie werden durch Sulfonierung mit Chlorsulfonsäure der entsprechenden 2-[2′H-Benzotriazol-(2′)-yl]-phenole hergestellt, wobei diese Ausgangsstoffe gemäss dem Verfahren von J. Rosevear und J.F.K. Wilshire, Aust. J. Chem 1985, 38, 1163-1176 hergestellt werden. Besonders die Herstellung der für die Herstellung der 3-[2′H-Benzotriazol-(2′)-yl]-4-hydroxybenzolsulfonate benötigten Ausgangsstoffe hat sich als schwierig und unwirtschaftlich erwiesen (vgl. l.c. Seite 1169, Verbindung D).

Es wurde nun ein Verfahren gefunden, welches die Herstellung von 3-[2′H-Benzotriazol-(2′)-yl]-4-hydroxybenzolsulfonsäuren und deren Salzen in einfacher Weise und in sehr guten Ausbeuten erlaubt.

Das erfindungsgemässe Verfahren zur Herstellung von 3-[2′H-Benzotriazol-(2′)-yl]-4-hydroxybenzolsulfonsäuren und deren Salzen der Formel

(1) 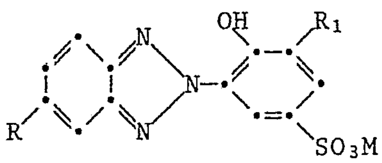

worin
R Wasserstoff oder Chlor,
$R_1$ verzweigtes oder geradkettiges $C_1$-$C_{12}$-Alkyl oder Phenyl-$C_1$-$C_3$-alkyl und
M Natrium oder Kalium bedeuten,
ist dadurch gekennzeichnet, dass man ein 2-(2′-Hydroxy-5′-tert.-butylphenyl)-benzotriazol der Formel

(2) 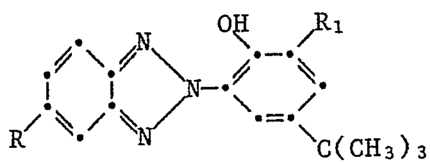

worin R und $R_1$ die oben angegebene Bedeutung haben mit Schwefelsäure sulfiert, wobei die tert.-Butylgruppe unter Abspaltung von Isobutylen durch die Gruppe $-SO_3M$ ersetzt wird, und das erhaltene Endprodukt auf pH 7 neutralisiert.

Die für die Sulfierung benötigte Schwefelsäure kann als 80-100%-ige, vorzugsweise 96-100%-ige Schwefelsäure oder vorzugsweise als Oleum bis zu einem Gehalt von 25 % freiem $SO_3$ zur Anwendung kommen.

Die Reaktionstemperaturen können in weiten Grenzen schwanken, z.B. zwischen 0 und 200°C. Verwendet man Schwefelsäure-monohydrat, so werden Temperaturen von 70 bis 100°C, besonders 80 bis 90°C bevorzugt. Verwendet man dagegen 25%-iges Oleum, so sind Temperaturen von 10 bis 30°C angezeigt. Die Reaktion kann gegebenenfalls bei leicht vermindertem Druck z.B. 146-187 mbar durchgeführt werden.

Bedeutet $R_1$ verzweigtes oder geradkettiges $C_1$-$C_{12}$-Alkyl, so kann es sich um die Gruppen Methyl, Ethyl, Propyl, iso-Propyl, Butyl, iso-Butyl oder sek.-Butyl, sowie um die Gruppen Pentyl, Hexyl, Heptyl, Octyl, Nonyl, Decyl, Undecyl, Dodecyl oder deren Isomere handeln. $R_1$ ist vorzugsweise ein verzweigtes $C_4$-$C_{12}$-Alkyl wie sek.-Butyl, 1-Methylbutyl, -pentyl, -hexyl, -heptyl-, -octyl, -nonyl-, decyl oder -undecyl. $R_1$ ist ganz bevorzugt sek.-Butyl.

Bedeutet $R_1$ einen Phenyl-$C_1$-$C_3$-alkylrest, so handelt es sich um den Benzyl-, Phenethyl- oder $\alpha,\alpha$-Dimethylbenzylrest.

Die erfindungsgemäss herstellbaren Verbindungen der Formel (1) können zur fotochemischen Stabilisierung von natürlichen und synthetischen Fasern verwendet werden (vgl. EP-A-0 112 120).

Die folgenden Beispiele dienen zur Erläuterung der Erfindung.

Beispiel 1:
80,9 g 2-(2′-Hydroxy-3′-sek.-butyl-5′-tert.butylphenyl)-benzotriazol werden innerhalb von einer Stunde bei 15-20°C in 150 ml 25%igem Oleum eingetragen. Es entsteht eine Lösung, die noch 16 Stunden bei

Raumtemperatur gerührt wird. Anschliessend lässt man die Lösung in ein Gemisch aus 600 g Eis und 400 ml Wasser unter gutem Rühren einfliessen. Das ausgefallene Produkt wird auf 80°C erhitzt und nach dem Abkühlen auf Raumtemperatur abfiltriert. Die Säure wird gut abgepresst und dann in einem Liter Wasser ausgeschlämmt. Danach wird unter Rühren mit 30%iger Natronlauge in 1 $\frac{1}{2}$ Stunden neutralisiert (pH 7). Der dick ausgefallene Kristallbrei wird nun erneut auf 80°C erhitzt, wobei eine gut filtrierbare Kristallform entsteht, und nach dem Abkühlen bei Raumtemperatur abfiltriert. Er wird bei 100°C im Vakuum getrocknet. Man erhält 83,5 g (90,4 % d. Th.) der Verbindung der Formel

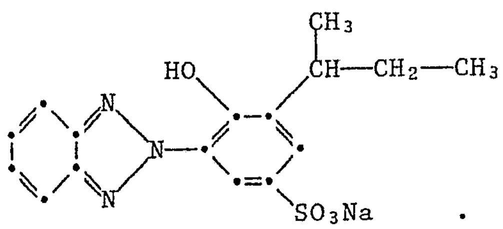

.

Beispiel 2:

161,5 g 2-(2′-Hydroxy-3′-sek.butyl-5′-tert.butylphenyl)-benzotriazol werden unter Rühren innerhalb von 15 Minuten in 640 g Schwefelsäure-Monohydrat eingetragen, wobei die Temperatur auf 35°C ansteigt. Unter leichtem Vakuum (117-143 mbar) wird anschliessend in einer Stunde auf 80°C erhitzt und noch vier Stunden bei dieser Temperatur gerührt. Nach dem Abkühlen auf 60°C lässt man die dunkle Lösung in 2 Liter Wasser von 50°C einfliessen, wobei die Temperatur auf 85°C ansteigt. Es wird noch 20 Minuten bei 80-85°C gerührt, dann auf Raumtemperatur abgekühlt und die ausgefallene Säure abfiltriert. Die Säure wird dann in 1 Liter Wasser angeschlämmt und mit 30%-iger Natronlauge auf pH 7 gestellt. Das Natriumsalz wird abfiltriert und mit 400 ml 3%-iger wässriger Natriumchloridlösung gewaschen. Nach dem Trocknen bei 80°C im Vakuum erhält man 172 g (93,1 % d. Th.) des Produktes der in Beispiel 1 angegebenen Formel.

Beispiel 3:

161,5 g 2-(2′-Hydroxy-3′-sek.-butyl-5′-tert.-butylphenyl)-benztriazol werden unter Rühren innerhalb von 5 Minuten in 350 ml 92%-iger Schwefelsäure eingetragen, wobei die Temperatur von 20 auf 26°C ansteigt. Unter Leichtem Vacuum wird die orangerote Lösung innerhalb von 90 Minuten auf 80°C erhitzt und noch 15 Stunden bei dieser Temperatur gerührt. Nach dem Abkühlen auf 60°C lässt man die Lösung in eine auf 50°C erwärmte Lösung von 100 g Kochsalz in 2 liter Wasser einfliessen. Dabei steigt die Temperatur auf 84°C an. Es wird 15 minuten bei dieser Temperatur gerührt und nach dem Abkühlen auf 40°C wird die ausgefallene Säure abfiltriert und anschliessend in 1 Liter Wasser angeschlämmt. Danach wird mit 30%-iger Natronlauge auf pH 7 gestellt. Das Natriumsalz wird abfiltriert und mit 400 ml 30 %-iger wässriger Natriumchloridlösung gewaschen. Nach dem Trocknen bei 80°C im Vacuum erhält man 168 g (92 % d.Th.) des Produktes der in Beispiel 1 angegebenen Formel.

**Patentansprüche**

1. Verfahren zur Herstellung von 3-[2′H-Benzotriazol-(2′)-yl]-4-hydroxybenzolsulfonsäuren und deren Salzen der Formel

(1) 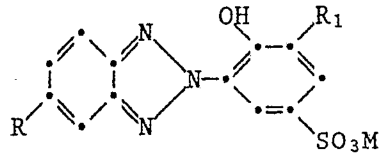

worin

R Wasserstoff oder Chlor,

$R_1$ verzweigtes oder geradkettiges $C_1$-$C_{12}$-Alkyl oder Phenyl-$C_1$-$C_3$-alkyl und

M Natrium oder Kalium bedeuten,
dadurch gekennzeichnet, dass man ein 2-(2′-Hydroxy-5′-tert.-butylphenyl)-benzotriazol der Formel

(2) 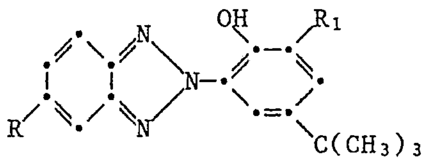

worin R und $R_1$ die oben angegebene Bedeutung haben mit Schwefelsäure sulfiert, wobei die tert.-Butylgruppe unter Abspaltung von Isobutylen durch die Gruppe -$SO_3$M ersetzt wird, und das erhaltene Endprodukt auf pH 7 neutralisiert.

2. Verfahren gemäss Anspruch 1 zur Herstellung von Verbindungen der in Anspruch 1 angegebenen Formel, worin $R_1$ Methyl, Ethyl, Propyl, iso-Propyl, Butyl, iso-Butyl oder sek.-Butyl, sowie um die Gruppen Pentyl, Hexyl, Heptyl, Octyl, Nonyl, Decyl, Undecyl, Dodecyl oder deren Isomere bedeutet.

3. Verfahren gemäss Anspruch 1 zur Herstellung von Verbindungen der in Anspruch 1 angegebenen Formel, worin $R_1$ sek.-Butyl, 1-Methylbutyl, -pentyl, hexyl, -heptyl-, -octyl, -nonyl-, decyl oder -undecyl bedeutet.

4. Verfahren gemäss Anspruch 1 zur Herstellung der Verbindung der in Anspruch 1 angegebenen Formel worin R Wasserstoff und $R_1$ sek.-Butyl bedeuten.

5. Verfahren gemäss einem der Ansprüche 1 bis 4, dadurch gekennzeichnet, dass man als Schwefelsäure 80-100%ige Schwefelsäure oder Oleum bis zu einem Gehalt von 25 % freiem $SO_3$ verwendet.

6. Verfahren gemäss einem der Ansprüche 1 bis 5, dadurch gekennzeichnet, dass die Sulfonierung bei einer Temperatur zwischen 0 und 200° C vorgenommen wird.

7. Verfahren gemäss einem der Ansprüche 1 bis 6, dadurch gekennzeichnet, dass die Sulfonierung bei einem leicht reduzierten Druck vorgenommen wird.

Europäisches Patentamt

# EUROPÄISCHER RECHERCHENBERICHT

Nummer der Anmeldung

EP 88 81 0714

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | GB-A-1 396 107 (CIBA-GEIGY)<br>* Insgesamt, insbesondere Seite 4, Beispiel 4 *<br>--- | 1-7 | C 07 D 249/20 |
| A | JOURNAL OF THE CHEMICAL SOCIETY, Perkin Transactions II, Nr. 5, März 1985, Seiten 677-682, London, GB; H.J.A. LAMBRECHTS et al.: "Aromatic sulphonation. Part 93. Sulphonation of the threet-butylphenols, four di-t-butylphenols, and 2,4,6-tri-t-butylphenol"<br>* Insgesamt *<br>----- | 1-7 | |
| | | | **RECHERCHIERTE SACHGEBIETE (Int. Cl.4)** |
| | | | C 07 D 249/00 |

Der vorliegende Recherchenbericht wurde für alle Patentansprüche erstellt

| Recherchenort | Abschlußdatum der Recherche | Prüfer |
|---|---|---|
| DEN HAAG | 19-01-1989 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)